Europäisches Patentamt

European Patent Office

Office européen des brevets

(11) Publication number: 0 356 724
A2

# (12) EUROPEAN PATENT APPLICATION

(21) Application number: 89114028.7

(22) Date of filing: 29.07.89

(51) Int. Cl.5: C12N 5/02

(30) Priority: 29.07.88 US 226037

(43) Date of publication of application:
07.03.90 Bulletin 90/10

(84) Designated Contracting States:
CH DE FR GB IT LI SE

(71) Applicant: BIOCHEM TECHNOLOGY INC.
66 Great Valley Parkway Great Valley
Corporate Center
Malvern Pennsylvania 19355(US)

(72) Inventor: Ju, Lu-Kwang
634 Summit House
West Chester PA 19382(US)
Inventor: Lee, Jaw Fang
25 Radnor Drive
Newtown Square, PA 19073(US)
Inventor: Armiger, William B.
21 Woodstream Drive
Wayne PA 19087(US)

(74) Representative: Weber, Dieter, Dr. et al
Dr. Dieter Weber und Dipl.-Phys. Klaus
Seiffert Patentanwälte
Gustav-Freytag-Strasse 25 Postfach 6145
D-6200 Wiesbaden 1(DE)

(54) Method of increasing the quantity of product produced by fragile cell and tissue cultures.

(57) A process in which a multiplicity of in vitro fragile cells are grown in a reaction medium contained within a reactor vessel for production of a biological product from said cells, a method for increasing the quantity of product produced by improving contact between cells and the medium to encourage cell growth comprises the steps of:
selecting a reaction medium capable of sustaining and nurturing said cells;
introducing said medium and said cells into said vessel;
determining the tendency of said cells to rise within said medium or sink within said medium;
selecting a non-toxic additive capable of alternating the tendency of said cells to rise or sink;
calculating the quantity of said additive necessary to effect a change in said tendency to rise or sink; and
introducing a quantity of said additive into said medium to alter said tendency to a desired degree to improve continuous cell suspension within said medium, thereby increasing non-turbulent, low-shear cell contact with nutrients in said medium to enhance cell growth and product production.

# METHOD OF INCREASING THE QUANTITY OF PRODUCT PRODUCED BY FRAGILE CELL AND TISSUE CULTURES

## FIELD OF THE INVENTION

This invention relates to improved methods of suspending, protecting, and supplying oxygen to cells in cultivation systems of fragile cells and tissues.

## BACKGROUND OF THE INVENTION

Cell cultures are used industrially to produce vaccines and certain therapeutic compounds. The importance of cell cultures is clearly indicated as new products derived from mammalian cells are introduced and the growing market sizes of these products are realized. Interferons, plasminogen activators, monoclonal antibodies, migration inhibition factors, tumor antigens, angiogenic factors, and certain hormones are examples of emerging products produced by cell cultures.

There are two types of mammalian cells; anchorage-dependent cells (cells that require a surface for growth) and suspension cells (nonanchorage-dependent). The traditional method used for large-scale production of anchorage-dependent cells employs the insides of rotating bottles as the surface area for growth. Rolling action of the bottles ensures that the cells are alternately exposed to growth medium and oxygen in the air space. This method is very cumbersome and expensive for production of large quantities of cells. Many roller bottles are needed to produce even a small number of cells since the surface area for growth is only a small percentage of the total bottle volume. Thus, roller bottles require extensive handling, labor, and a large quantity of medium to produce a high quantity of cells. In addition, cells produced from a series of roller bottles are highly variable between separate cultures.

The variation in cell cultures makes monitoring of cellular kinetics and the ability to change the growth environment to obtain optimal production rates practically impossible. To increase the ratio of the surface area available for cell growth to the total culture volume, current technology employs microcarrier beads suspended in liquid culture media for cultivation of anchorage-dependent cells. Therefore, both anchorage-dependent cells and suspension cells can be cultivated in suspension systems similar to those used in the fermentation industry.

To increase production of mammalian cell products on a large scale, it is desirable to maximize the cell concentration obtained in a cell culture. Higher cell concentration results in an increased quantity of "biological catalysts", and a higher product concentration. Costs of purification of the product decreases as product concentration increases. Furthermore, no additional serum components are needed at higher cell concentrations, since serum components are thought to catalyze cellular reactions and are not depleted to a great extent in the medium. Therefore, at higher cell concentrations, the unit yield of product per unit of serum used increases. High cell concentrations greatly reduce the cost of producing mammalian cell products since serum is the predominant cost of culture media.

To achieve high cell concentrations and, thus, to maximize product yield in large-scale cell cultures, cells should be suspended within the medium and supplied with ample oxygen. Systems and technology are available for large-scale fermentation of microbial organisms. However, without adequate modifications, they tend not to be suitable for optimal cell culture growth. In the absence of rigid cell walls which protect microorganisms, high-shear agitation and/or high-rate air sparging used to suspend and supply oxygen to cells in conventional fermentors is sufficient to damage the fragile plasma membrane of cells, leading to metabolic changes and cell death.

Most contemporary industrial systems use either stirred-tank or column (airlift) bioreactors for large-scale cell cultures for both anchorage-dependent and suspension cells. In stirred-tank bioreactors, suspension of cells and oxygenation are typically achieved by mechanical agitation and surface or direct sparge aeration, individually or in combination. In small-scale cultivation systems, mild agitation with surface aeration is usually sufficient for cell suspension and oxygenation. However, the maximum shear rate induced by such agitation is proportional to the impeller tip speed, $\pi NDi$, where $N$ and $Di$ are the agitation speed and the impeller diameter, respectively. As the system scale increases, the suspending and oxygen supply capabilities of the bioreactor decrease due to lower agitation speed.

In column bioreactors, on the other hand, air sparging is typically the sole source of cell suspension and oxygenation of the system. A rather high air sparging rate must be applied to homogeneously suspend the cells in the column. Experimental results reported in the literature on cell culture in column bioreactors has shown that oxygen limitation has not been reached in the studied systems. The highest cell concentrations achievable in these studies were probably limited by damage to the cells caused by shear associated with high air-sparging rates. Therefore, better methods of suspending, protecting, and supplying oxygen to cells are essential to cell culture product production.

## SUMMARY OF THE INVENTION

The present invention provides a method of improving suspension of fragile cells and tissues in in vitro cultivation systems by changing the density difference between cells or tissues and their surrounding environment and increasing the apparent viscosity of the culture medium. The first component alters the driving force for cells and tissues to settle or rise in the systems, while the second component substantially decreases the settling or rising velocity of cells and tissues. By applying this method in cell and tissue cultures, high energy input, which tends to generate high shear rates in the systems, for suspension of cells and tissues is reduced.

Another aspect of the invention shows that perfluorocarbon emulsions protect cells from deadly effects of excessive shear. While the exact mechanism for the protective effect of the perfluorocarbon emulsions is unknown, it is believed that oil-in-water emulsion droplets attach to the gas-liquid interface and protect the cells from being entrained in the circulating gas-liquid interface at the gas bubble surface, and carried at high velocities in the film until bubble break-up occurs. Perfluorocarbon emulsions can thus be used in cultivation systems of fragile cells as cell-protective agents.

A mathematical model has been developed in accordance with aspects of this invention to describe changes in oxygen transfer rates in cell culture systems upon addition of oil-in-water emulsions. The change in oxygen transfer rate in an aerobic bioreactor has been shown to depend strongly on not only the oxygen solubility of the non-aqueous phase, but also the oxygen diffusion coefficient of the non-aqueous phase and the change in hydrodynamic property of the system due to the presence of the oil-in-water emulsion.

## BRIEF DESCRIPTION OF THE FIGURES

Figure 1 summarizes in table format experimental results of total cell concentration and cell viability obtained in the first run of hybridoma culture made in column bioreactors, in accordance with aspects of the invention and two column bioreactors in accordance with conventional methods.

Figure 2 graphically shows experimental results of total and viable cell concentrations in samples taken from top and bottom sampling lines of the conventional reactor methods disclosed in Figure 1.

Figure 3 graphically shows experimental results of total and viable cell concentrations in samples taken from top and bottom sampling lines of the reactor methods of the invention as disclosed in Figure 1.

Figure 4 summarizes in table format experimental results of total cell concentration and cell viability obtained in the second run of hybridoma culture made in two column bioreactors, in accordance with aspects of the invention and two column bioreactors in accordance with conventional methods.

Figure 5 shows the composition of medium used.

Figure 6 shows the experimental results of dissolved oxygen and viable cell concentration obtained.

## DETAILED DISCUSSION OF THE INVENTION

In cultivation systems of suspension cells or tissues (hereinafter sometimes collectively referred to as "cells" for the sake of brevity), when no agitation and aeration are involved, cells settle due to their higher density than that of the aqueous media. Settling velocity is estimated by equating the force of gravity on cells with the buoyancy force and the drag forces, i.e.,

$$V = \frac{D_c^2 (d_c - d) g}{18\mu} \qquad (1)$$

where $D_c$ is the equivalent diameter of cell, g is the gravitational acceleration, $\mu$ is the viscosity of the medium, and $d_c$ and $d$ are densities of cell and medium, respectively.

3

In culture systems of anchorage-dependent cells, on the other hand, cells are attached to microcarrier beads and, thus, settle or rise with the beads according to the difference between the effective density of the microcarrier beads with attached cells and the density of the culture medium. While settling or rising velocity can still be estimated by the above equation, $D_c$ and $d_c$ in this case represent the equivalent diameter and the effective density of a microcarrier bead with the attached cells, respectively.

Based on the above-mentioned equation, a method of improving the cell-suspending ability, i.e., lowering the velocity of cell settling or rising, has been discovered by increasing the apparent viscosity of the medium and/or decreasing density differences between the culture medium and the suspension cells or the microcarrier beads with attached anchorage-dependent cells. The former is accomplished by introducing medium apparent viscosity enhancers to the systems and the latter by making the overall medium density about the same as that of cells or microcarrier beads with attached cells via addition of medium effective density modifiers. For cultivation of anchorage-dependent cells and tissues, an alternative method for reducing the density difference is to use microcarrier beads of different densities.

While substances which increase the apparent viscosity of the medium by forming true solutions, emulsions, solid-liquid suspensions, or other formulations with the culture medium may be employed in accordance with the method of the invention, perfluorocarbon emulsions and polymers, including proteins, polysaccharides, polysaccharide-derivatives, other biopolymers, and synthetic polymers, are preferred. Many polymers dissolve in aqueous solutions and increase the solution viscosities dramatically with only trace amounts of polymers present. They are thus preferably used as cell-suspending agents in large-scale stirred-tank bioreactors and column bioreactors.

Another advantage of using these apparent viscosity enhancers in cell and tissue cultures is that by controlling the viscosity of the culture media, the cells are protected from excessive shear. However, in assessing the role of medium apparent viscosity enhancers in cell and tissue cultures, it should be noted that the overall oxygen transfer coefficient, $k_La$, depends very strongly on the viscosity of liquid medium. Thus, in cultivation systems where the overall rate of oxygen intake is controlled by physical absorption, increases in medium viscosity tend to result in reduction of the overall reaction rate and maximum cell concentration achievable.

It is expected that different cell lines have different densities. However, they all tend towards about 1.1 $g/cm3$ and are higher than densities of ordinary aqueous media, i.e., about 1.0 $g/cm3$. In the case of cell and tissue cultures of anchorage-dependent cells with microcarrier beads, the effective density of beads with attached cells is usually higher than that of the liquid medium, since microcarrier beads mostly comprise polymers having densities larger than the densities of aqueous media. One effective way of suspending cells is to increase the medium effective density to about the same as that of the suspension cells or the microcarrier beads with cells so that there is either none or only a slight tendency for cells to settle or rise.

Many substances can modify the medium density by forming true solutions, emulsions, solid-liquid suspension or other formulations with the culture medium. An emulsion of FC-40 FLUORINERT® Electronic Liquid (3M, St. Paul, MN, a perfluorocarbon with an average molecular weight of 650 and a density of 1.87 $g/cm3$, hereinafter referred to as "FC-40"), in an aqueous culture medium with a density of 1.0 $g/cm3$ is a preferred example. The volume fraction of FC-40 needed to make the effective medium density the same as that of a suspension cell line with a density of 1.1 $g/cm3$ can be estimated as:

1.87 x + 1.0 (1 - x) = 1.1

i.e. x = 11.49%

Experimental results of hybridoma cell culture in column bioreactors using this emulsion as the cell-suspending agent are described hereinafter.

Hybridoma 14-4-4S, ATCC HB32, was used as the sample cell line for this study, due to its well-documented optimal growth environments, monoclonal antibody production rate, and specific oxygen uptake rate. Derived by fusing SP2/O-Ag14 cells with lymphocytes of C3H cells, the cell line produces a cytotoxic monoclonal antibody (IgG$_{2a}$K) that reacts with the I-E$_k$ and C$_k$ determinants.

The base medium used in the study was supplemented Dulbecco's Modified Eagles Medium (DMEM) with glutamine and low glucose. The medium was further supplemented with the following substances, added per liter of DMEM: 3.7 g sodium bicarbonate, 10 ml non-essential amino acids, 100,000 units penicillin, 100 mg streptomycin, 13.2 mg oxaloacetic acid, 0.8 mg insulin, 5.5 mg pyruvic acid, 3.0 g glucose, and 110 ml fetal bovine serum.

Hybridoma culture was made in four column bioreactors, hereinafter R1, R2, R3, and R4, simultaneously, The columns had dimensions of 60 cm in length and 6.52 cm in diameter, and working volumes of 1.8 liters. Each bioreactor was equipped with a pH electrode and a dissolved oxygen electrode. pH values were controlled to range from 6.9 to 7.3 by altering the partial pressure of $CO_2$ in the gas stream. The experiment was performed in a temperature-controlled room with the temperature well maintained at 37 ±

4

0.1 °C. A draft tube was suspended in R1 to make an airlift bioreactor.

An oil-in-water emulsion comprising 40% (volume to volume) perfluorocarbon FC-40 and 60% (volume to volume) aqueous solution of 5% (weight to volume) PLURONIC® F-68 (BASF Wyandotte Corporation, Parsippany, NJ, a non-ionic surfactant having a mean molecular weight of about 8,350) was made by emulsifying the two immiscible phases with a MICROFLUIDIZER® M-110 (Microfluidics Corporation, Newton, MA). The emulsion was then diluted with concentrated culture medium to make at 11% in volume FC-40 emulsion in which the aqueous phase is the same as the predescribed, normal culture medium. The 11% FC-40 emulsion thus prepared was used in R3 and R4 in accordance with the invention, while the medium in R1 and R2 contained no perfluorocarbon emulsion. In order to assess how well the cells were suspended, three sample lines were included in each column to collect sample from top, middle, and bottom parts of the bioreactor, respectively. Cell numbers were determined by the trypan blue exclusion method using a haemocytometer.

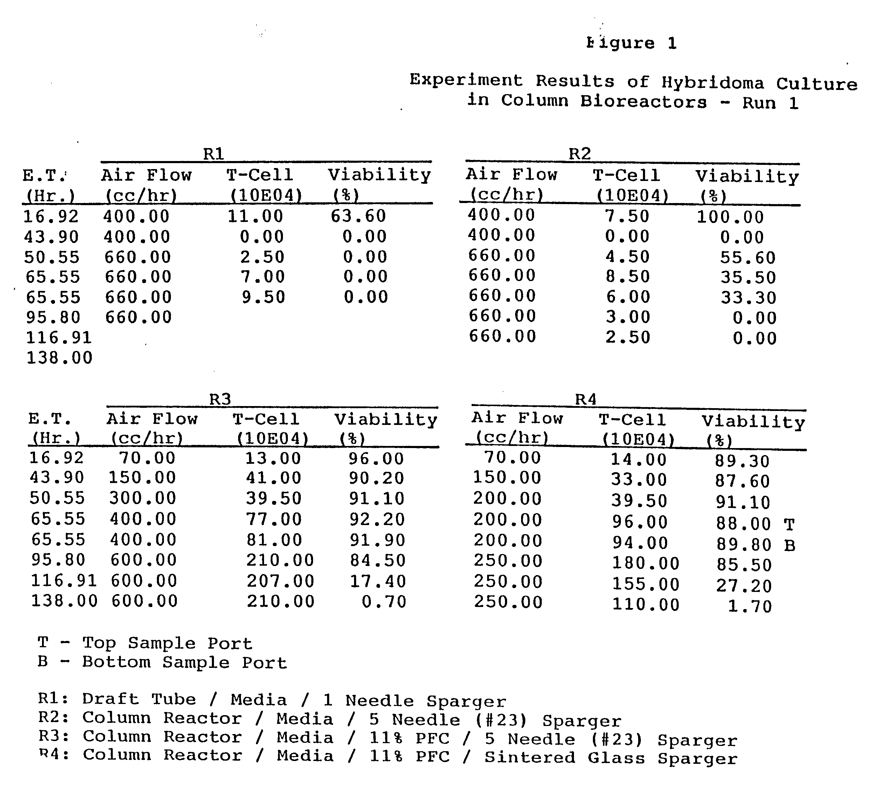

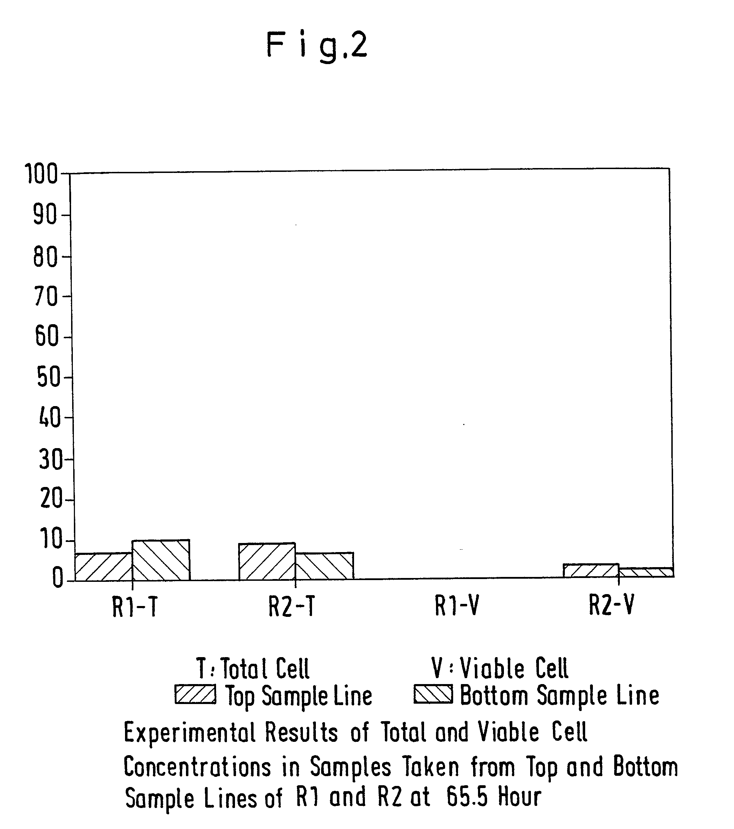

Experimental results are summarized in Figure 1. The gas flow rate of 400 ml/hr, i.e., 0.22 vvh, was initially used in R1 and R2, where no perfluorocarbon emulsions were present. Cells settled to the bottom plate of the bioreactor are about 50 hours. The gas flow rate was then increased to 660 ml/hr, i.e., 0.37 vvh, to suspend the cells. At this gas flow rate, however, cells were severely physically damaged by increased shear induced by the gas bubbles. Figure 2 shows experimental results of total and viable cell concentrations in samples taken from top and bottom sampling lines of R1 and R2 at 65.55 hr (conventional method).

On the other hand, the initial gas flow rate used in R3 and R4 was only 70 ml/hr, i.e., 0.039 vvh. It was then increased gradually to meet the increased oxygen requirement of the cells as they grew. Nevertheless, no cell sedimentation was observed throughout the run.

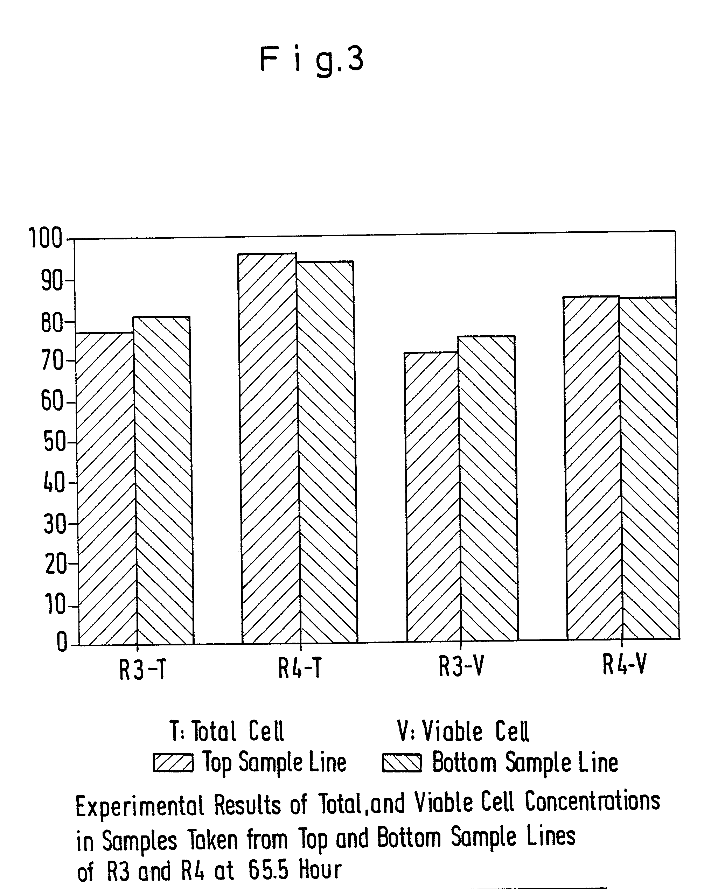

Figure 3 shows results of total and viable cell concentrations in samples taken from top and bottom sampling lines of R3 and R4 at 65.55 hr. While the gas flow rates at that time are 400 ml/hr and 200 ml/hr in R3 and R4 respectively, no appreciable difference was noticed. This clearly indicates that cells were well suspended in the medium with 11% FC-40 emulsion, due to the fact that the overall density of the emulsion is about the same as that of hybridoma cells.

As shown in Figure 2, shear forces cause damage to fragile cells. Obtaining a method of cell protection is thus very valuable to obtaining viable cell cultures. While there have been indications in the art that polymer additives provide at least some degree of protection of cells from excessive shear, the inventors have discovered that oil-in-water type emulsions have the ability to protect cells from shear. The protection mechanism of polymers is believed to be due to the increase in apparent liquid viscosity. Since emulsions also raise the apparent liquid viscosity, depending on the ratio of the volume of inner to outer phase, droplet size, size distribution, and viscosities of inner and outer phases, similar effects of cell protection apply to cell culture systems with emulsions. In addition, experimental results (described hereinafter) indicate the perfluorocarbon emulsions tend to stabilize foam at the surface of the medium. The inventors believe that oil-in-water emulsion droplets attach to the gas-liquid interface in the medium and protect the cells from being entrained in the circulating gas-liquid interface at the gas bubble surface, and carried at high velocities in the film until bubble break-up occurs.

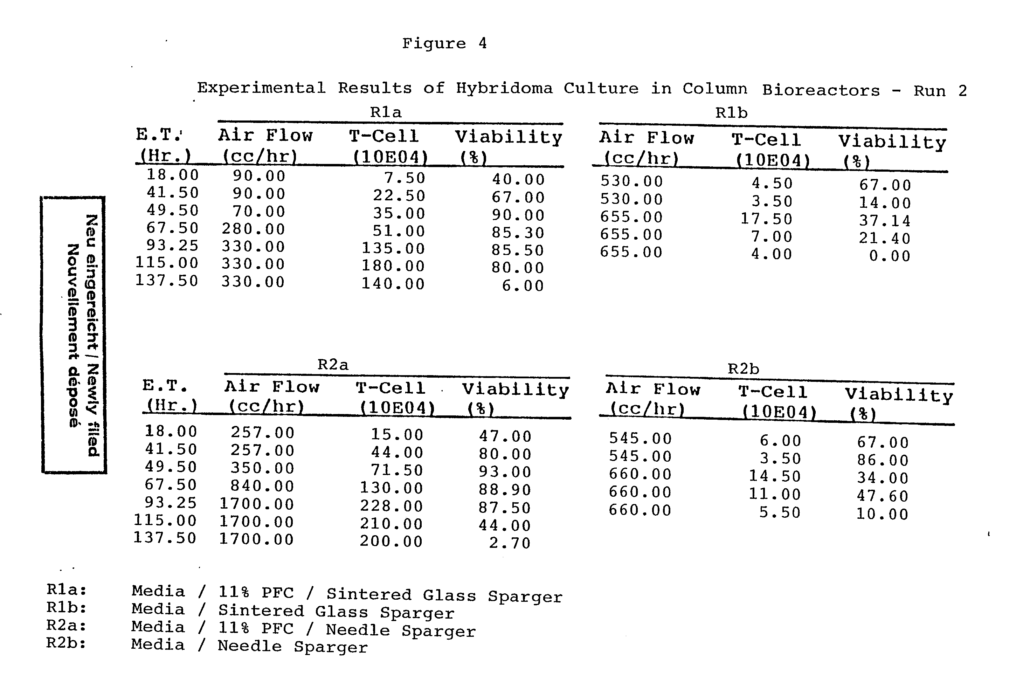

To study the cell protective effect of perfluorocarbon emulsions, a repeated run of hybridoma cell culture in column bioreactors was made. Figure 4 shows the experimental results obtained. While all the previous observations were maintained, the gas flow rate in R2a where 11% of FC-40 emulsion was applied, were gradually increased to as high as 840 ml/hr, i.e., 0.47 vvh, without noticeable damage to the cells. Cell damage caused by shear induced by gas bubbles was observed only after the gas flow rate had been further increased to an extraordinarily high value of 1700 ml/hr i.e., 0.94 vvh. The cell protective effect of 11% FC-40 emulsion was clearly shown when compared to the severe cell damage observed at gas flow rate of the lower value of 660 ml/hr in bioreactors without the emulsion.

The maximum viable cell concentration achieved in bioreactors supplied with FC-40 emulsion was $2.3 \times 10^6$ cells/ml, while that in bioreactors without FC-40 emulsion never reached $1 \times 10^5$ cells/ml.

The use of emulsified perfluorinated compounds as artificial blood substitutes is known in the art. Such artificial bloods utilize the emulsions as oxygen carriers. While it is mainly the thermodynamic property of high oxygen solubilities in these perfluorinated compounds that attracts the attention of others, our interest in applying emulsions in cell culture systems partially rests on the kinetic properties of oxygen transfer rates in the bioreactors. Thus, emulsions utilized in accordance with the present invention serve not as oxygen carriers but as oxygen transfer enhancers.

Oxygen supply has been a central topic for discussion and scale-up of aerobic bioprocesses. It plays and even more important role in cell and tissue cultures. To avoid damage to fragile mammalian cells and tissues, only mild agitation and/or low-rate air sparging can be applied in cell and tissue culture bioreactors. The overall oxygen supply process in bioreactors is controlled by the liquid-film resistance of mass transfer through gas-liquid interface. The oxygen transfer rate, OTR, can be expressed as:

$$OTR = k_L a (CO_2^* - CO_2)$$
$$= k_L a\, S\, (P_g - P_1) \qquad (3)$$

where $k_L$, $a$, $CO_2^*$, $CO_2$ are liquid-phase oxygen transfer coefficient, interfacial area per unit volume of medium, saturation concentration of dissolved oxygen at the gas-liquid interface, and bulk concentration, respectively; and $S$, $P_g$, and $P_1$ are oxygen solubility, in terms of Henry's Law Constant, of the medium, and oxygen partial pressure of the gas phase and that in the bulk medium, respectively.

The effects of additives on oxygen transfer rate in the culture system can be described as the oxygen-transfer enhancement factor, E, defined as the ratio of oxygen transfer rates through the gas-liquid interface with and without the presence of the additives. By combining equation (3) and

$$k_L = (Ds)^{1/2} \qquad (4)$$

the oxygen-transfer enhancement factor can be expressed as

$$E = \left(\frac{D_{eff}}{D_o}\right)^{1/2} \left(\frac{S_{eff}}{S_o}\right)^{1/2} \left(\frac{S_{eff}}{S_o}\right) \qquad (5)$$

although the true situation would be more complicated. Equation (4) is the result of the surface-renewal theory derived by Danckwerts in 1951. D is oxygen diffusion coefficient, s is surface-renewal rate, i.e., the fraction of liquid surface renewed per unit time, and subscripts eff and o represent effective and original values with and without the presence of these additives, respectively.

As shown in equation (5), in addition to the effective oxygen solubility of the culture medium with the emulsion, the oxygen-transfer enhancement factor depends strongly on changes in the effective oxygen diffusion coefficient and hydrodynamic conditions upon addition of these additives.

Although experimental results of oxygen diffusion coefficients in perfluorocarbons (PFCs) have been scarcely reported, they are believed to be larger than or of the same order of magnitude as the value in pure water. For rough, conservative estimates of E, we assumed that $D_{eff}/D_o \sim 1$. $S_{eff}$ is a volume-averaged property, i.e.,

$$S_{eff} = S_o\,\phi_o + S_{PFC}\,\phi_{PFC}$$
$$= S_o\,[1 + (m - 1)\,\phi_{PFC}] \qquad (6)$$

where $m = S_{PFC}/S_o$, $\qquad (7)$

and $\phi$ is the volume fraction. An approximate relationship between E and the volume fraction of PFC emulsions present can be expressed as:

$$E \sim \left(\frac{S_{eff}}{S_o}\right)^{1/2} [1 + (m - 1)\,\phi_{PFC}] \qquad (8)$$

In systems of very dilute PFC emulsions, the assumption of no appreciable change of surface-renewal rate, s, upon addition of the emulsions is valid, and a linear relationship was developed as:

$$E \sim 1 + (m - 1)\phi_{PFC} \qquad (9)$$

Nonetheless, as mentioned above, emulsion-forming generally raises the apparent liquid viscosity, depending upon the ratio of the volume of inner to outer phase, droplet size, size distribution, and viscosities of inner and outer phases, and, thus, decreases the surface-renewal rate, s. The best formulation for oxygen-transfer enhancers used in cell culture systems is far different from that applied in artificial blood substitutes.

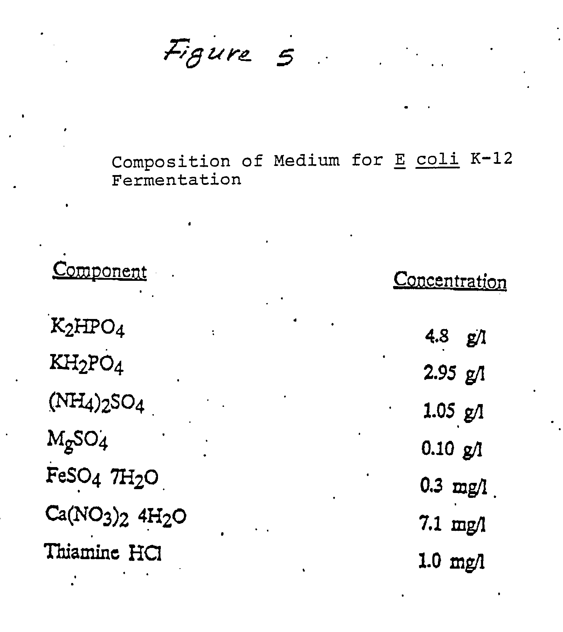

To demonstrate the enhancement in oxygen transfer rate in bioreactors via introduction of oxygen-carrying particles, fermentations of E. coli K-12 were conducted in two 1-liter double sidearm, water jacketed Celstir (Wheaton Scientific, Millville, NJ) simultaneously. Each bioreactor was equipped with one pH electrode and one dissolved oxygen electrode. The agitation speed was 100 rpm and the temperature was controlled at $37 \pm 0.1°$C. The base medium composition employed in the fermentations is listed in Figure 5. While the medium in one reactor contained at 15% emulsion of FC-77 FLUORINERT® Electronic Liquid (hereinafter referred to as "FC-77"), that in the other was the base medium. FC-77 is a perfluorocarbon manufactured by 3M, St. Paul, MN, having average molecular weight of 415. Samples were taken periodically and plated in petri dishes containing agar for viable cell counts. The final sample of each bioreactor was taken 5 minutes after the dissolved oxygen content dropped to zero.

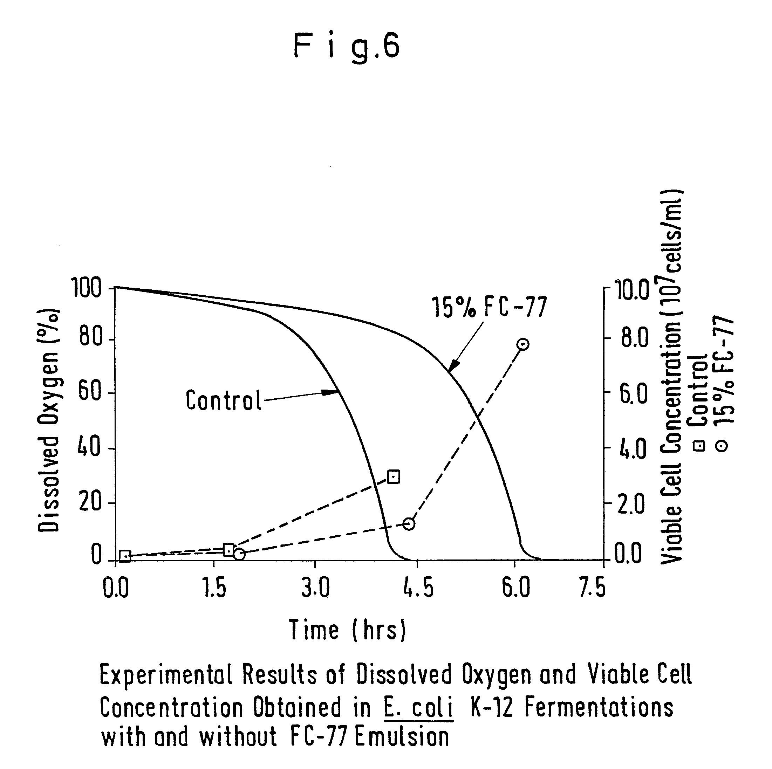

Experimental results are shown in Figure 6. It is apparent that the dissolved oxygen content dropped much slower in the bioreactor with 15% FC-77 emulsion than in the other. The enhancement of oxygen transfer rate through introduction of 15% FC-77 emulsion is clearly shown. Thus, use of perfluorocarbon emulsions enhances oxygen transfer in addition to providing cell suspension and cell protection.

As shown in equation (3), the maximum oxygen transfer rate achievable in a bioreactor is reached when the dissolved oxygen content in the culture medium drops to zero, i.e., $C_{O2} = P_1 = 0$. By assuming the peudo-steady state of oxygen transfer rate equal to the oxygen consumption rate, the maximum oxygen transfer rate, $(OTR)_{max}$, can be estimated as the product of specific oxygen demand of the interested organism and the viable cell concentration at $C_{O2} = P_1 = 0$. With the assumption of the same specific oxygen demand of cells in bioreactors with and without PFC emulsions, the oxygen-transfer enhancement factor, E, in the studied system by applying 15% FC-77 emulsion can be calculated as the ratio of the viable cell concentrations in samples taken when the dissolved oxygen content dropped down to zero, i.e.,

$$E = \frac{7.9 \times 10^7 \ (cells/ml)}{3.1 \times 10^7 \ (cells/ml)} = 2.55 \qquad (10)$$

The value of 2.55 is smaller than the value of 3.55 calculated by using equation (9) for oxygen-transfer enhancement factor in a system containing 15% FC-77 emulsion. This is mainly attributed to the lower surface-renewal rate in the system with FC-77 emulsion. The necessity of considering the overall transport property of PFC emulsions used in cell culture as oxygen-transfer enhancers rather than only oxygen solubility when used as artificial blood substitutes is clearly demonstrated.

Although the foregoing description has been directed somewhat specifically to application of PFC emulsions in cell and tissue cultures, the specification is not intended to limit the invention to the particular embodiments described hereinabove, but various modifications may be made therein and thereto without departing from the scope and spirit of the invention as set forth in the following claims.

## Claims

1. In a process in which a multiplicity of in vitro fragile cells are grown in a reaction medium contained within a reactor vessel for production of a biological product from said cells, a method for increasing the quantity of product produced by improving contact between cells and the medium to encourage cell growth comprising of the steps of:
selecting a reaction medium capable of sustaining and nurturing said cells;
introducing said medium and said cells into said vessel;
determining the tendency of said cells to rise within said medium or sink within said medium;
selecting a non-toxic additive capable of alternating the tendency of said cells to rise or sink;
calculating the quantity of said additive necessary to effect a change in said tendency to rise or sink; and
introducing a quantity of said additive into said medium to alter said tendency to a desired degree to improve continuous cell suspension within said medium, thereby increasing non-turbulent, low-shear cell contact with nutrients in said medium to enhance cell growth and product production.

2. The method according to claim 1 wherein said cells are aggregated as tissues.

3. The method according to claim 1 wherein said additive is selected from the group consisting of perfluorocarbons, perfluorocarbon emulsions, proteins, polysaccharides, polysaccharide derivatives, hydrocarbons, hydrocarbon emulsions and gums.

4. The method according to claim 1 wherein said additive alters the apparent viscosity of said medium.

5. The method according to claim 1 wherein said additive alters the effective density of said medium.

6. The method according to claim 4 wherein the quantity of additive introduced into said medium substantially eliminates the tendency of said cells to rise or sink.

7. The method according to claim 5 wherein the quantity of additive introduced into said medium substantially eliminates the tendency of said cells to rise or sink.

8. The method according to claim 1 wherein said additive is an emulsion of a perfluorocarbon having a density of about 1.87 g/cm³.

9. The method according to claim 8 wherein about 11-12% by volume of said emulsion is added.

10. The method according to claim 1 wherein said cells are carried by microcarriers.

11. The method according to claim 10 wherein said microcarriers have the selected density to substantially eliminate the tendency of said cells carried by said microcarriers to rise or sink.

12. The method according to claim 10 wherein said cells are aggregated as tissues.

13. In a process in which a multiplicity of in vitro fragile cells are grown in a reactor vessel containing a reaction mixture having a reaction medium and wherein the reaction mixture is agitated to facilitate bulk mixing of said reaction mixture and transfer of reactant gas to said cells and wherein said agitation subjects said cells to high shear stress and increases cell collisions, the improvement comprising protecting said cells from said high shear stress and said collisions by introducing a perfluorocarbon emulsion into said vessel, wherein said emulsion adheres to at least a portion of the surface of reactant gas bubbles within said agitated mixture, thereby reducing direct contact between said bubbles and said cells to decrease shear stress applied to said cells and decrease direct collisions between said cells.

14. The improvement defined in claim 13 wherein said emulsion assists in transfer of reactant gas to said medium.

15. The improvement defined in claim 13 wherein said emulsion decreases collisions between cells carried by microcarriers.

16. The improvement defined in claim 13 wherein said perfluorocarbon emulsion is an emulsion of a perfluorocarbon having a density of about 1.5-2.0 g/cm$^3$ and an average molecular weight of about 400-1000.

17. The improvement defined in claim 16 wherein about 5-25% by volume of said emulsion is added.

EP 0 356 724 A2

## Figure 1

### Experiment Results of Hybridoma Culture in Column Bioreactors – Run 1

| | R1 | | | R2 | | |
|---|---|---|---|---|---|---|
| E.T. (Hr.) | Air Flow (cc/hr) | T-Cell (10E04) | Viability (%) | Air Flow (cc/hr) | T-Cell (10E04) | Viability (%) |
| 16.92 | 400.00 | 11.00 | 63.60 | 400.00 | 7.50 | 100.00 |
| 43.90 | 400.00 | 0.00 | 0.00 | 400.00 | 0.00 | 0.00 |
| 50.55 | 660.00 | 2.50 | 0.00 | 660.00 | 4.50 | 55.60 |
| 65.55 | 660.00 | 7.00 | 0.00 | 660.00 | 8.50 | 35.50 |
| 65.55 | 660.00 | 9.50 | 0.00 | 660.00 | 6.00 | 33.30 |
| 95.80 | 660.00 | | | 660.00 | 3.00 | 0.00 |
| 116.91 | | | | 660.00 | 2.50 | 0.00 |
| 138.00 | | | | | | |

| | R3 | | | R4 | | |
|---|---|---|---|---|---|---|
| E.T. (Hr.) | Air Flow (cc/hr) | T-Cell (10E04) | Viability (%) | Air Flow (cc/hr) | T-Cell (10E04) | Viability (%) |
| 16.92 | 70.00 | 13.00 | 96.00 | 70.00 | 14.00 | 89.30 |
| 43.90 | 150.00 | 41.00 | 90.20 | 150.00 | 33.00 | 87.60 |
| 50.55 | 300.00 | 39.50 | 91.10 | 200.00 | 39.50 | 91.10 |
| 65.55 | 400.00 | 77.00 | 92.20 | 200.00 | 96.00 | 88.00 T |
| 65.55 | 400.00 | 81.00 | 91.90 | 200.00 | 94.00 | 89.80 B |
| 95.80 | 600.00 | 210.00 | 84.50 | 250.00 | 180.00 | 85.50 |
| 116.91 | 600.00 | 207.00 | 17.40 | 250.00 | 155.00 | 27.20 |
| 138.00 | 600.00 | 210.00 | 0.70 | 250.00 | 110.00 | 1.70 |

T - Top Sample Port
B - Bottom Sample Port

R1: Draft Tube / Media / 1 Needle Sparger
R2: Column Reactor / Media / 5 Needle (#23) Sparger
R3: Column Reactor / Media / 11% PFC / 5 Needle (#23) Sparger
R4: Column Reactor / Media / 11% PFC / Sintered Glass Sparger

EP 0 356 724 A2

# F i g.2

T : Total Cell          V : Viable Cell

▨ Top Sample Line     ◩ Bottom Sample Line

Experimental Results of Total and Viable Cell
Concentrations in Samples Taken from Top and Bottom
Sample Lines of R1 and R2 at 65.5 Hour

Neu eingereicht / Newly filed
Nouvellement déposé

# Fig.3

T: Total Cell    V: Viable Cell

▨ Top Sample Line    ▨ Bottom Sample Line

Experimental Results of Total,and Viable Cell Concentrations
in Samples Taken from Top and Bottom Sample Lines
of R3 and R4 at 65.5 Hour

## Figure 4

Experimental Results of Hybridoma Culture in Column Bioreactors - Run 2

| E.T. (Hr.) | R1a Air Flow (cc/hr) | T-Cell (10E04) | Viability (%) | R1b Air Flow (cc/hr) | T-Cell (10E04) | Viability (%) |
|---|---|---|---|---|---|---|
| 18.00 | 90.00 | 7.50 | 40.00 | 530.00 | 4.50 | 67.00 |
| 41.50 | 90.00 | 22.50 | 67.00 | 530.00 | 3.50 | 14.00 |
| 49.50 | 70.00 | 35.00 | 90.00 | 655.00 | 17.50 | 37.14 |
| 67.50 | 280.00 | 51.00 | 85.30 | 655.00 | 7.00 | 21.40 |
| 93.25 | 330.00 | 135.00 | 85.50 | 655.00 | 4.00 | 0.00 |
| 115.00 | 330.00 | 180.00 | 80.00 | | | |
| 137.50 | 330.00 | 140.00 | 6.00 | | | |

| E.T. (Hr.) | R2a Air Flow (cc/hr) | T-Cell (10E04) | Viability (%) | R2b Air Flow (cc/hr) | T-Cell (10E04) | Viability (%) |
|---|---|---|---|---|---|---|
| 18.00 | 257.00 | 15.00 | 47.00 | 545.00 | 6.00 | 67.00 |
| 41.50 | 257.00 | 44.00 | 80.00 | 545.00 | 3.50 | 86.00 |
| 49.50 | 350.00 | 71.50 | 93.00 | 660.00 | 14.50 | 34.00 |
| 67.50 | 840.00 | 130.00 | 88.90 | 660.00 | 11.00 | 47.60 |
| 93.25 | 1700.00 | 228.00 | 87.50 | 660.00 | 5.50 | 10.00 |
| 115.00 | 1700.00 | 210.00 | 44.00 | | | |
| 137.50 | 1700.00 | 200.00 | 2.70 | | | |

R1a: Media / 11% PFC / Sintered Glass Sparger
R1b: Media / Sintered Glass Sparger
R2a: Media / 11% PFC / Needle Sparger
R2b: Media / Needle Sparger

EP 0 356 724 A2

*Figure 5*

Composition of Medium for E coli K-12
Fermentation

| Component | Concentration |
|---|---|
| $K_2HPO_4$ | 4.8 g/l |
| $KH_2PO_4$ | 2.95 g/l |
| $(NH_4)_2SO_4$ | 1.05 g/l |
| $MgSO_4$ | 0.10 g/l |
| $FeSO_4\ 7H_2O$ | 0.3 mg/l |
| $Ca(NO_3)_2\ 4H_2O$ | 7.1 mg/l |
| Thiamine HCl | 1.0 mg/l |

# Fig.6

Experimental Results of Dissolved Oxygen and Viable Cell
Concentration Obtained in E. coli K-12 Fermentations
with and without FC-77 Emulsion